# EUROPEAN PATENT SPECIFICATION

(11) **EP 2 753 335 B1**
(45) Date of publication and mention of the grant of the patent: **11.01.2017**
(21) Application number: 12780554.7
(22) Date of filing: 07.09.2012
(51) Int. Cl.: A61K 31/704, A61P 29/00

(54) **AN ANTHRACYCLINE FOR THE TREATMENT OF SEPSIS**
EIN ANTHRACYCLIN ZUR BEHANDLUNG VON SEPSIS
UNE ANTHRACYCLIN POUR LE TRAITEMENT DE SEPSIS

(30) Priority: 08.09.2011 PT 11105881
(43) Date of publication of application: 16.07.2014
(73) Proprietor: Instituto de Medicina Molecular, 1649-028 Lisboa (PT); Ferreira Moita, Luis, 1750-413 Lisboa (PT)
(72) Inventor: FERREIRA MOITA, Luis, P-1750-413 Lisboa (PT)
(74) Representative: Pereira da Cruz, Jorge Afonso
(86) International application number: PCT/PT2012/000034
(87) International publication number: WO 2013/036153

(56) References cited:
- US-A1- 2003 139 353
- KRYSKO D V ET AL: "TLR-2 and TLR-9 are sensors of apoptosis in a mouse model of doxorubicin-induced acute inflammation", CELL DEATH AND DIFFERENTIATION, NATURE PUBLISHING GROUP, GB, vol. 18, no. 8, 1 August 2011 (2011-08-01) , pages 1316-1325, XP009164954, ISSN: 1350-9047, DOI: 10.1038/CDD.2011.4
- SAUTER KRISTIN A D ET AL: "Doxorubicin and daunorubicin induce processing and release of interleukin-1[beta] through activation of the NLRP3 inflammasome", CANCER BIOLOGY AND THERAPY, LANDES BIOSCIENCE, UNITED STATES, vol. 11, no. 12, 15 June 2011 (2011-06-15) , pages 1008-1016, XP009164952, ISSN: 1555-8576, DOI: 10.4161/CBT.11.12.15540
- CADEDDU C ET AL: "Protective effects of the angiotensin II receptor blocker telmisartan on epirubicin-induced inflammation, oxidative stress, and early ventricular impairment", AMERICAN HEART JOURNAL, MOSBY- YEAR BOOK INC, US, vol. 160, no. 3, 1 September 2010 (2010-09-01), pages 487.e1-487.e7, XP027256366, ISSN: 0002-8703 [retrieved on 2010-09-07]
- VAN DER MOST R G ET AL: "Decoding dangerous death: how cytotoxic chemotherapy invokes inflammation, immunity or nothing at all", CELL DEATH AND DIFFERENTIATION, NATURE PUBLISHING GROUP, GB, vol. 15, no. 1, 1 January 2008 (2008-01-01), pages 13-20, XP002538563, ISSN: 1350-9047, DOI: 10.1038/SJ.CDD.4402255 [retrieved on 2007-11-16]
- RITTIRSCH DANIEL ET AL: "Harmful molecular mechanisms in sepsis", NATURE REVIEWS IMMUNOLOGY, vol. 8, no. 10, October 2008 (2008-10), pages 776-787, XP002687830,

## Description

### FIELD OF THE INVENTION

The present invention relates to the field of medicaments for the treatment of sepsis, in particular severe sepsis, using anthracyclines, namely epirubicin, doxorubicin and daunorubicin.

### BACKGROUND OF THE INVENTION

Inflammation is a response to harmful stimuli that limits tissue damage and aims at restoring homeostasis⁴. Pathogen-associated molecular patterns (PAMPs) on microorganisms and damage-associated molecular patterns (DAMPs) originating from dying cells are sensed by the host through germline-encoded pattern recognition receptors (PRRs) that recognize conserved signature structures in non-self and self⁵⁻⁷. These sensors are present in both professional (including neutrophils, macrophages and dendritic cells) and nonprofessional immune cells and their activation initiates intracellular signaling cascades leading to the transcriptional expression of inflammatory mediators, such as cytokines and chemokines. Inflammation needs to be effectively terminated after removal of the original trigger and repair of damaged tissue. In the susceptible host, overproduction of inflammatory mediators or an exaggerated response to their presence can lead to septic shock, tissue destruction, permanent loss of function, immunodeficiency or autoimmunity⁸,

Sepsis is a life-threatening condition that arises as a systemic inflammatory response to an infection⁹. It is initiated by an overproduction of pro-inflammatory mediators potentially leading to decreased cardiac function, increased vascular permeability and metabolic unbalance ultimately causing multi-organ failure and death¹⁰. Sepsis includes a continuum of conditions ranging from systemic inflammatory syndrome (SIRS), which can progress to multi-organ failure and ultimately septic shock and death¹¹. Sepsis is the leading cause of death in intensive care units and the third cause of overall hospital mortality^{12,13}. The incidence of sepsis and its economic burden has increased in the past decades by 1% each year^{13,14}, and in spite of significant improvement in diagnosis and support measures, the mortality rates still range from less than 10% in SIRS to almost 80% in septic shock. The pathophysiology of sepsis remains poorly understood. As a result the basic elements of treatment (antibiotics, control of the source of infection and organ support) have not changed significantly in the last fifty years, and attempts to translate basic research results into effective new interventions have been met with limited or no success¹¹.

Sepsis is a heterogeneous and dynamic syndrome caused by immune dysfunction that might assume different aspects temporally and in different components of the immune system. For example, the early stage of sepsis is characterised by a hyper-inflammatory environment due to overwhelming activation of innate immune responses by infection or tissue damage, while later time points might be better described as an immunosuppressive state¹⁰. The early initiating step is dependent on tumor necrosis factor (TNF) and interleukin 1β (IL-1β) and the delayed, slow progressing phase, dependent on late mediators such as high mobility group box-1 (HMGB1)¹³.

Most often, sepsis is triggered by a bacterial infection that causes excessive production of pre-inflammatory mediators, including the initial critical TNF and IL-1β, leading to the activation of spiraling signaling cascades ultimately causing multi-organ failure and death¹⁰. We have performed chemical and genetic screens, which led us to the identification of the group of anthracyclines as potent inhibitors of their secretion and to the identification of ATM as a negative regulator of inflammation. Anthracyclines confer strong protection to the mouse model of sepsis by increasing the tolerance to the burden of infection, without increasing bacterial killing. At the molecular level, they inhibit the bacterially induced inflammatory program, in a partially ATM-dependent manner. ATM is strictly required for the in vivo protection against sepsis, which is based on the inhibition of inflammation mediators, the induction of autophagy and the protection of target-organ damage.

### BRIEF DESCRIPTION OF THE DRAWINGS

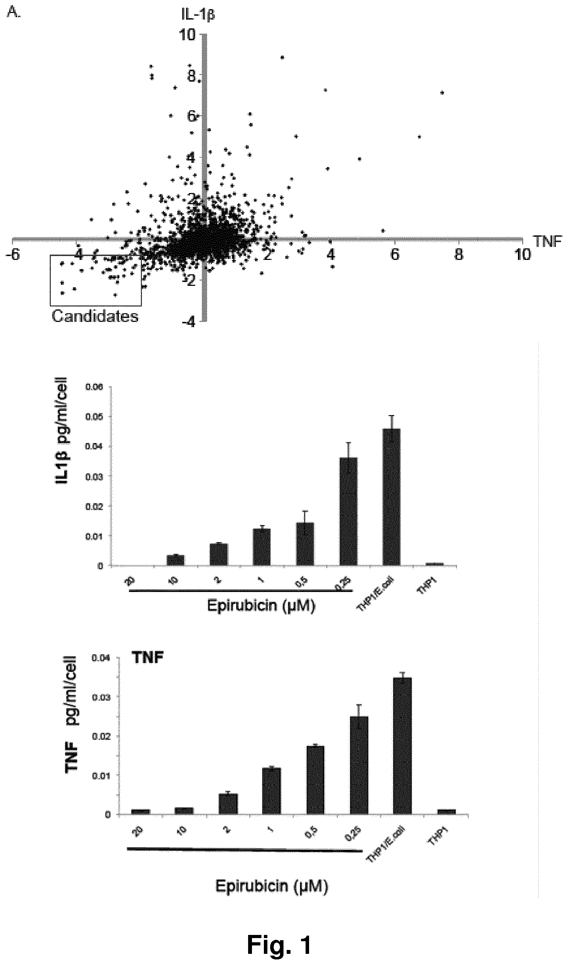

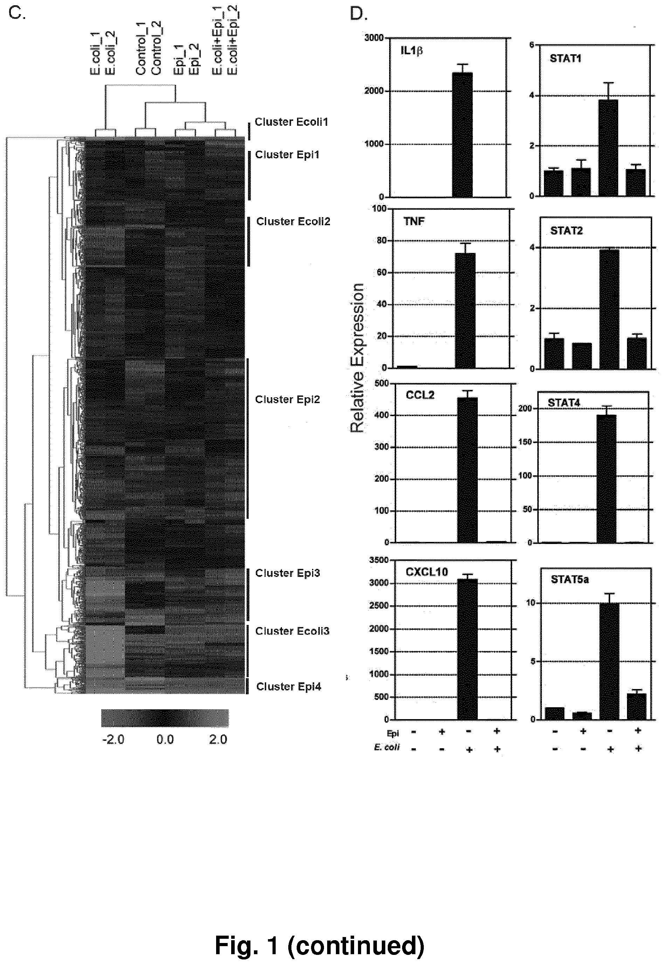

Figure 1 represents a primary screen to identify compounds in the Spectrum Collection library that inhibit the secretion of TNF and IL-1β. (A) it is a two-dimension plot of TNF and IL-1β production Z scores calculated upon THP-1 cells challenge with PFA-fixed *E. coli* for 24 hours in the presence of 10µM of each compound. The square defines the area in which compounds are considered primary hits, i.e., inhibiting both TNF and IL-1β, (B) represents the IL-1β and TNF production by *E*. *coli* challenged THP-1 cells (4 hours) after a pre-incubation (1 hour) with increasing concentrations of epirubicin. Results shown represent mean ± standard deviation from triplicate samples in one of 3 independent assays. (C) is a transcriptional profiling of THP-1 cell line response to epirubicin and unsupervised clustering of microarray expression profiles. Normalized nicroarray expression data from two replicate samples of untreated THP-1 cells (Control_1 and _2) and The-1 cells exposed to PFA-fixed *E. soli* (E.coli_1 and _2), epirubicin [Epi_₁ and _2) or both (E.coli+Epi_1 and _2) were used to perform a clustering analysis after filtering genes with either low expression or low variation across samples. Color gradient depicts variation in expression levels within a log2 range of -2 to 2 after mean centering gene expression values. GO classification of genes in selected clusters (Cluster Ecoli1 to 3 and Clutter Epi1 to 4) is indicated on the right. Vertical lines mark the location of the clusters in the gene tree. (D) relates to the RT-qPCR validation of microarray data. Plots depict relative expression of IL-1β, TNF, CCL2, CXCL10, STAT1, STAT2, START4 and STAT5a as assessed by qRT-PCR in *E*. *coli* challenged THP-1 cells (4 hours) after a pre-incubation (1 hour) with 5µM epirubicin. Results shown represent mean number of mRNA molecules of each gene per GAPDH mRNA molecule ± standard deviation from triplicate samples.

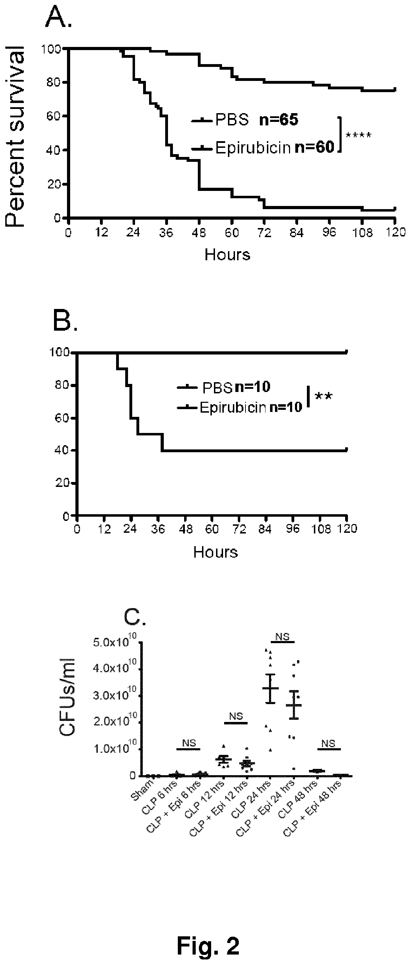

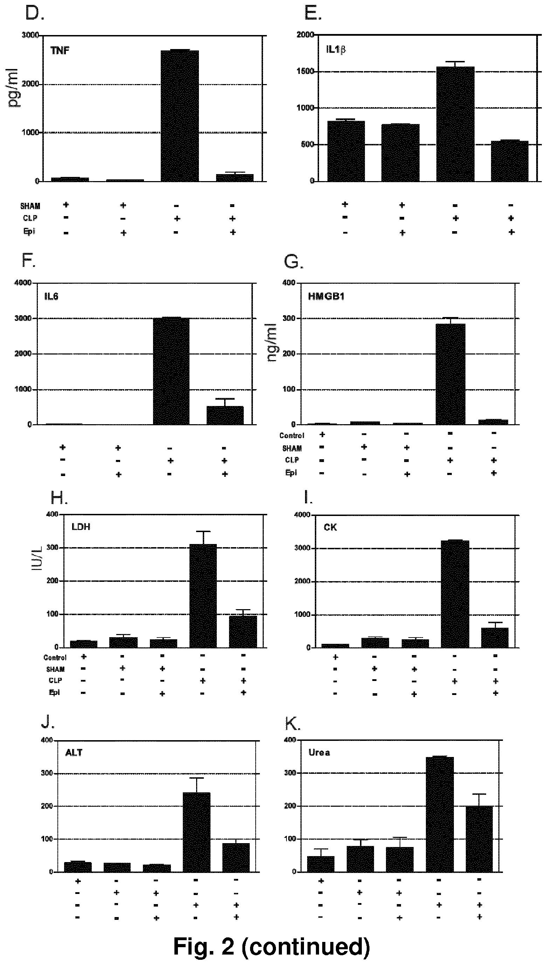

(Figure 2 shows that epirubicin affords protection against severe CLP. (A) and (B) relate to the survival of C57BL/6 wildtype animals subjected to (A) CLP or (B) lethal LPS injection treated with carrier (PBS) or epirubicin (0,6µg/g body weight) at the time of procedure and 2≤ hours later. NS, not significant; **P<0.01; ****P<0.0001 (log-rank (Mantel-Cox) test for 2A, 2B and Mann-Whitney test for 2C). (C) shows the polymicrobial load (CFUs) in the blood of C57BL/6 animals undergoing CLP at indicated time points. PBS and epirubicin treatment as before. Each circle represents individual animals. Bars are mean values ± SEM. Epirubicin counteracts inflammation and tissue damage associated with CLP. When indicated (+) C57HL/6 animals received PBS (n=3) or epirubicin (0.6µg/g body weight) (n=3) at 0 and 24 hours post CLP. (D) represents plasma concentrations of TNF, (E) of IL-1β, (F) of IL-6 and (G) of HMCB1 24 hours after CLP. Results shown represent mean values ± SEM from triplicate readings per animal. Measurements of (H) LDH, (I) CK, (J) ALT and (K) urea in plasma of C57BL/6 animals undergoing CLP and treated with the same dose and schedule as before are presented. Results shown represent mean values ± SEM from duplicate readings per animal (n=5).

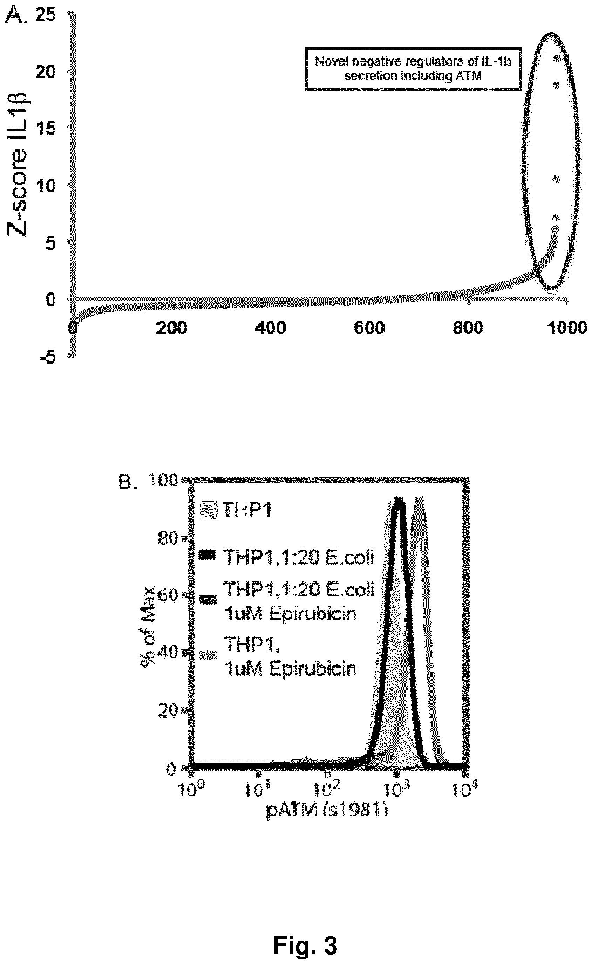

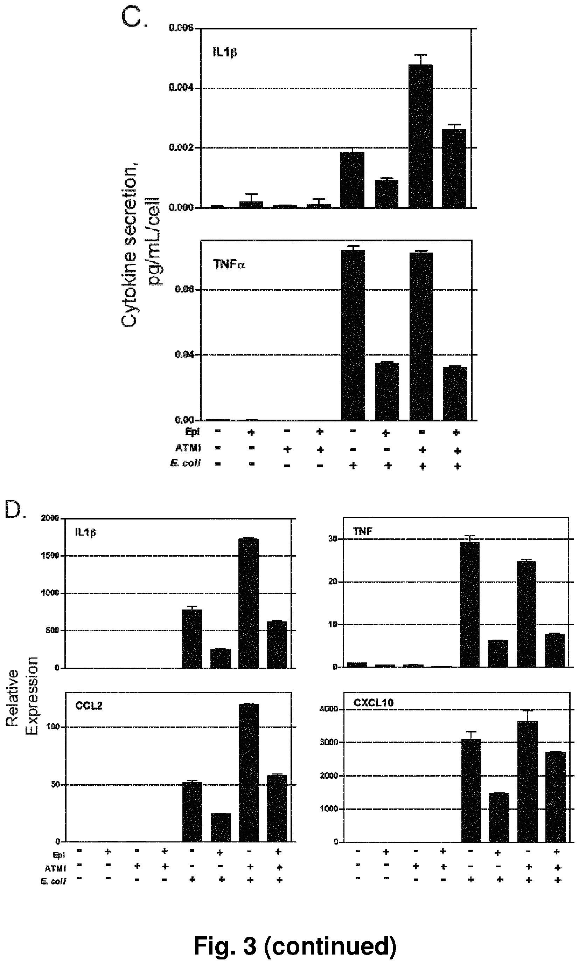

Figure 3 shows that anti-inflammatory effects of epirubicin are mediated by ATM. (A) is a Z score plot, sorted in ascending order, of IL-1β production by THP-1 cells upon target gene knockdown using a selected group of constructs of the TRC shRNA lentiviral vector library followed by PFA-fixed *E*. *coli* stimulation for 24 hours. Each dot represents an individual construct. The red circle defines the area in which compounds are considered primary hits. (B) refers to a flow cytometry analysis of the activated form of ATM, phosphorilated at serine 1981, in THP-1 cells left untreated or treated with epirubicin alone (1µM) (5 hours), challenged with PFA-fixed *E. coli* (4 hours) or *E. coli* (4 hours) plus epirubicin pre-treatment (1 hour). (c) represents the IL-1β and TNF production by *E. coli* challenged THP-1 cells (4 hours) after a pre-incubation (1 hour) with PBS, epirubicin, (1µM) or epirubicin plus KU-55933 (2µM) when indicated (+). Results shown represent mean ± standard deviation from triplicate samples in one of 3 independent assays. (D) relates to the expression of IL-1β, TNF, CCL2 and CXCL10 as assessed by qRT-PCR in THP-1 cells treated as in (C). Results shown represent mean number of mRNA molecules of each gene per GAPDH mRNA molecule ± standard deviation from triplicate samples.

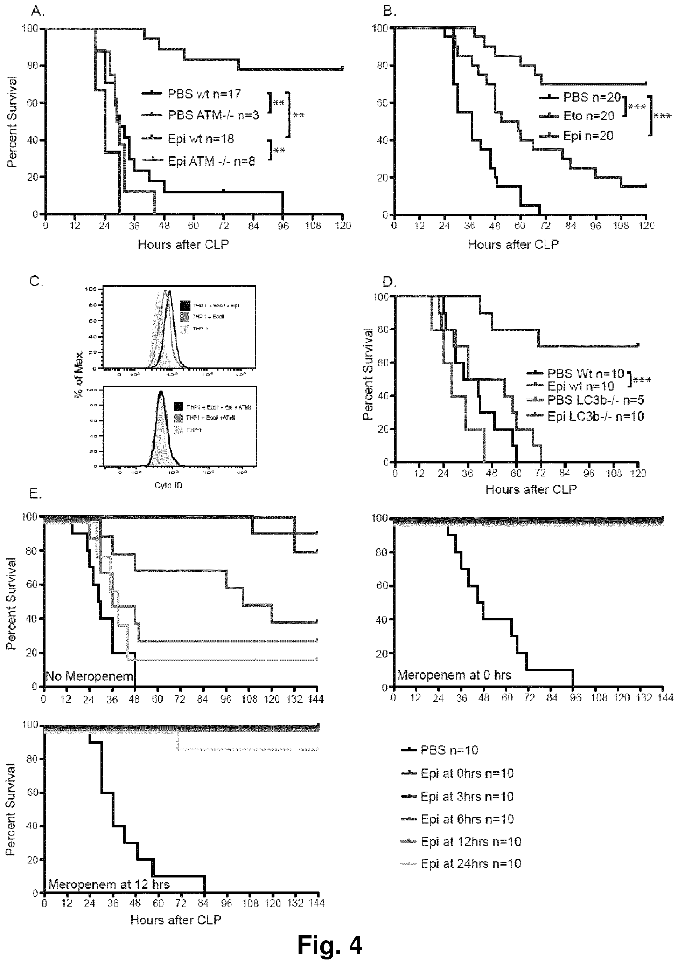

Figure 4 shows that ATM-dependent protection of epirubicin against CLP relies on the induction of autophagy. (A) represents the survival of wildtype and Atm^{-/-} C57BL/6 animals subjected to CLP and treated with PBS or epirubicin (0.6µg/g body weight) at the time of procedure and 24 hours later. (B) represents the survival of PBS-, etoposide-, and epirubicin-treated wildtype C57BL/6 animals undergoing CLP. Etoposide dose was 2.0µg/g body weight. Epirubicin dose and treatment schedule as if (A). (C) is a flow cytometry analysis of induction of autophagy in: (i) THP-1 cells left untreated or pre-treated with epirubicin (1µM) (1 hour) and challenged with PFA-fixed *E*. *coli* (4 hours) (upper histogram) or (ii) THP-1 cells pre-treated with KU-55933 (2µM) or epirubicin (IµM) plus KU-55933 (2µM) (1 hour) and challenged with PFA-fixed *E*. *soli* (4 hours) (lower histogram). (D) represents a survival of wildtype and LC3b^{-/-} C57BL/6 animals subjected to CLP and treated with PBS or epirubicin in the dose and schedule as in (A). *P<0.05; **p<0.01; ***P<0.001 (log-rank (Mantel-Cox) test). Epirubicin confers protection against severe CLP in a therapeutic manner. (E) relates to the urvival of C57BL/6 wildtype animals subjected to CLP treated with epirubicin (0-6µg/g body weight) at indicated time in the absence of meropenem (upper left panel); with a administration of meropenem (40µg/g body weight/day) starting at the time of the procedure (upper right panel) of with meropenem (40µg/g body weight/day) treatment starting 12 hours after CLP (lower left panel).

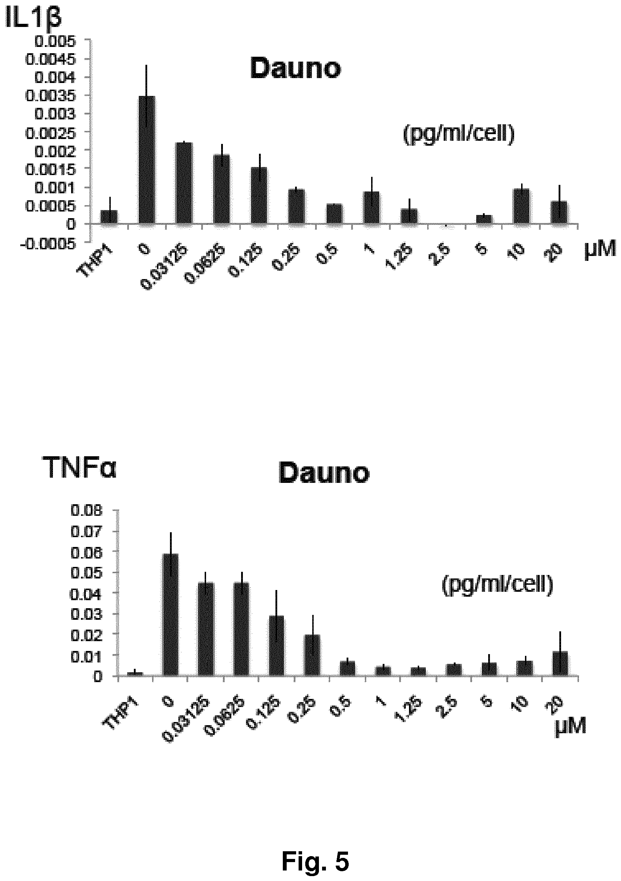

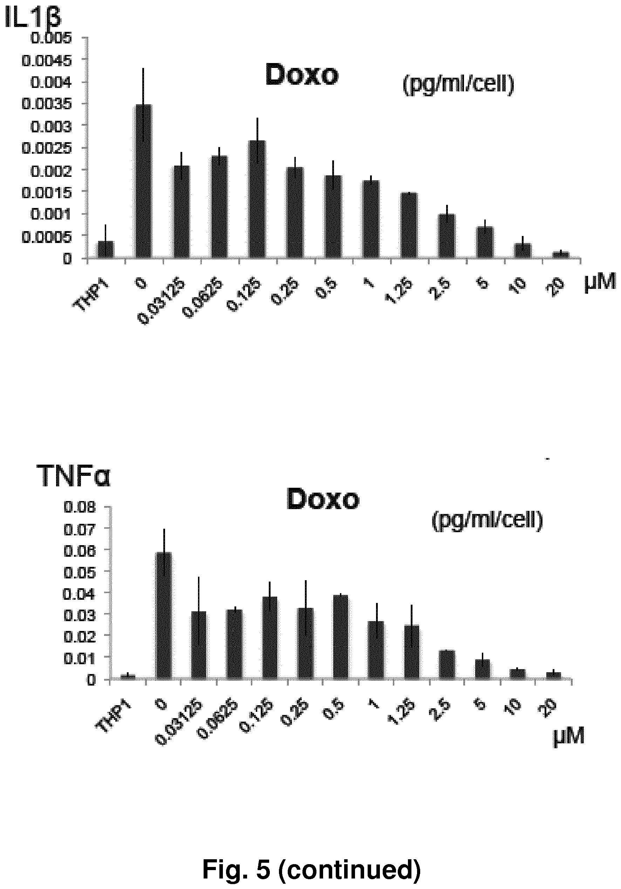

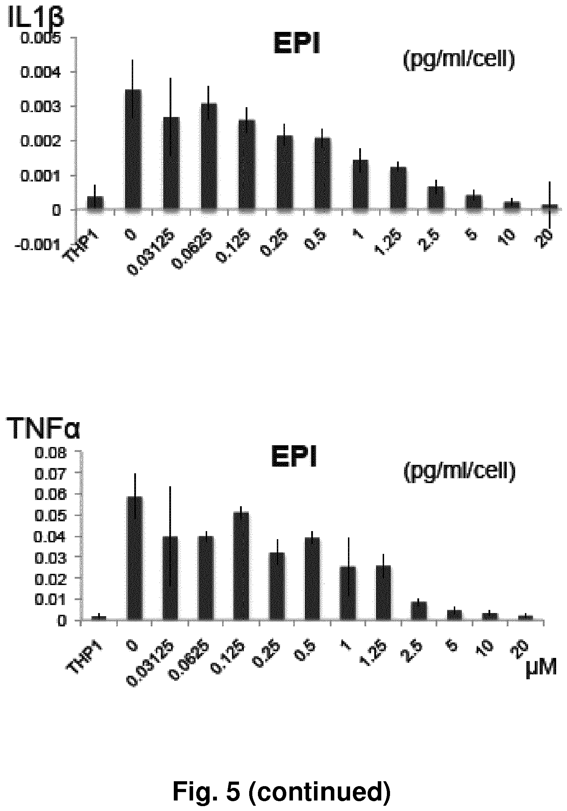

Figure 5 shows that antracyclines inhibit the secretion of TNF and IL-1β. IL-1β and TNF production by *E*. *coli* challenged The-1 cells (4 hours) after a pre-incubation (1 hour) with increasing concentrations of daunorubicin, doxorubicin and epirubicin. Results shown represent mean ± standard deviation from triplicate samples in one of 3 independent assays.

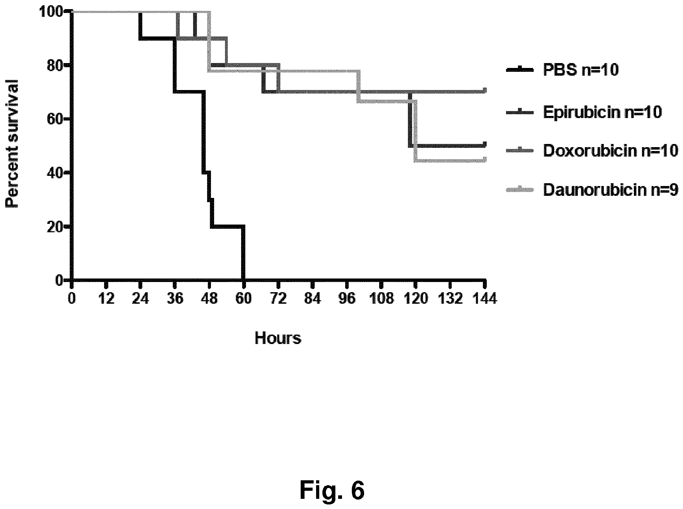

Figure 6 shows that other anthracycline, in addition to epirubicin, afford protection against severe CLP. Survival of C57BL/6 wildtype animals subjected to CLP treated with carrier (PBS), epirubicin (0.6µg/g body weight), doxorubicin (0.5ug/g body weight) or daunorubicin (0.6µg/g body weight) at the time of procedure and 24 hours later.

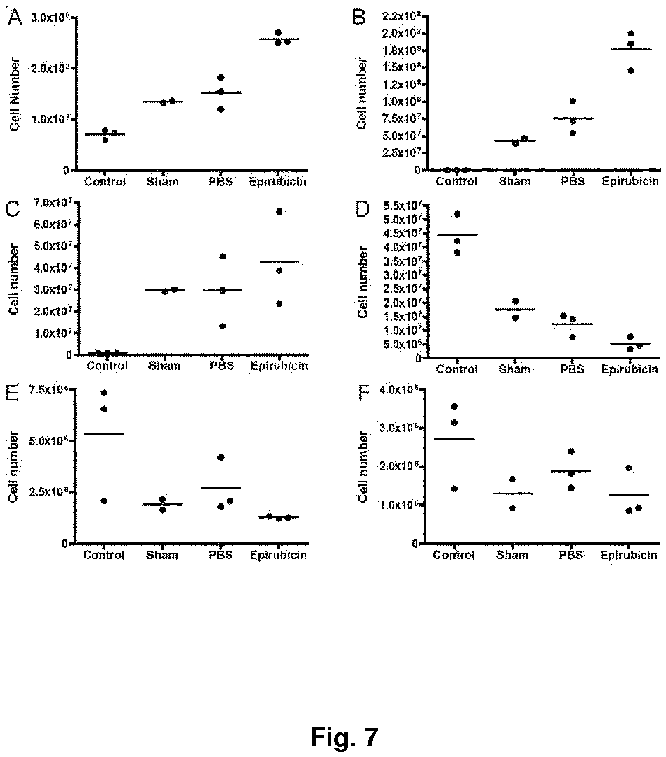

Figure 7 shows that epirubicin does not counteract inflammatory cell migration into the site of inflammation. Quantification of (A) total cells, (B) neutrophils, (C) macrophages, (D) B cells, (E) CD4 T and (F) CD8 T lymphocytes in the peritoneal cavity 18 hours post CLP of C57EL/6 wildtype animals treated with PBS or epirubicin (0.6µg/g body weight) at the time of procedure.

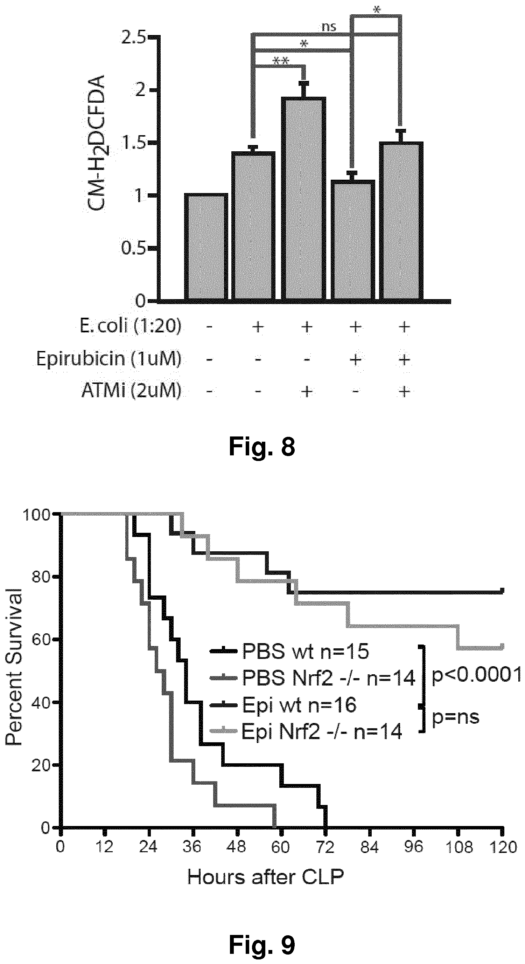

Figure 8 represents the production of free radicals in THP-1 cells as determined by flow cytometry with CM-H₂DCFDA. Cells were left untreated or when indicated (+) were pre-treated with epirubicin (1mM) (1 hour) or epirubicin (1mM) plus KU-55933 (2mM) (1 hour) and challenged with PFA-fixed E. coli (4 hours). Results shown represent control -normalized mean intensity of fluorescence values ± standard error of mean from three independent experiments.
Figure 9 refers to the survival of wildtype and Nrf2^{-/-} C57BL/6 animals subjected to CLP and treated with PBS or epirubicin (0.6µg/g body weight) at 0 and 24 hours post CLP.

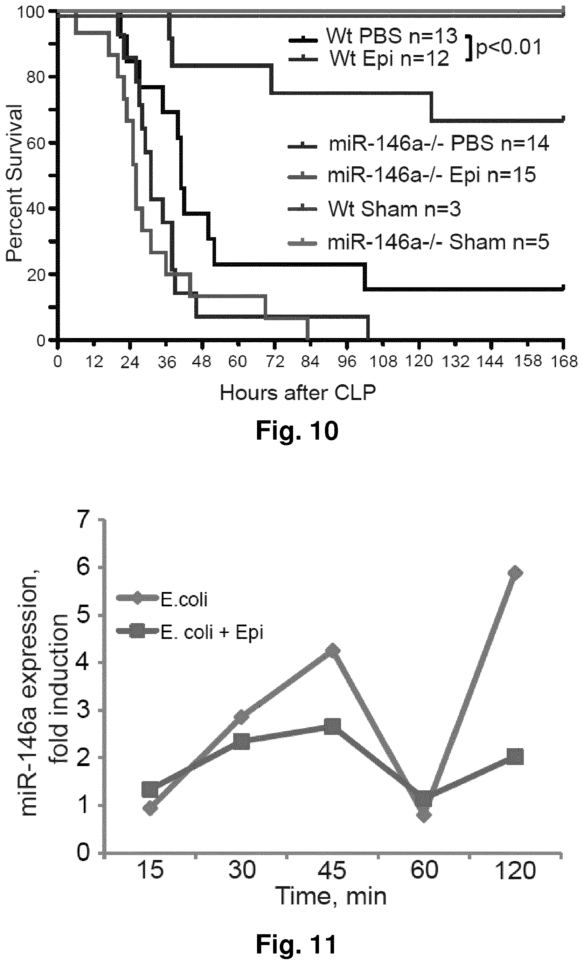

Figure 10 refers to the survival of wildtype and miR-145a^{-/-} C57BL/6 animals subjected to CLP and treated with PBS or epirubicin (0.6µg/g body weight) at 0 and 24 hours post CLP.
Figure 11 presents the time Course of miR-146a induction in response to epirubicin treatment of THP-1 cells challenged with PFA-fixed *E. coli,* as measured by RT-qPCR.

### SUMMERY OF THE INTENTION

Sepsis remains a poorly understood systemic inflammatory condition with high mortality rates and limited therapeutic options in addition to organ support measures¹. Most often, sepsis is triggered by a bacterial infection that causes excessive production of pro-inflammatory mediators, including the initial critical tumor necrosis factor (TNF) and interleukin 1β (IL-1β), leading to the activation of spiraling signaling cascades ultimately causing multi-organ failure and death^{2,3}. Here we used a drug screen to identify the clinically approved group of anthracyclines as potent in *vitro* inhibitors of two key initiators of sepsis, TNF and IL-1β. *In* vivo, anthracyclines, confer strong protection against severe sepsis induced by cecal ligation and puncture (CLP) in mice. This protective effect relies on the induction of autophagy and on an anti-inflammatory program that increase the tolerance to infection without reducing bacterial burden. Using an shRNA-based screen we identified the Ataxia Telangiectasia Mutated (ATM) as a mediator of the protective effect of anthracyclines. ATM deficient (Atm^{-/-} ) mice are refractory to this protective effect succumbing to severe sepsis with similar kinetics to the non-treated wild-type mice. Our results identify the group of anthracyclines as effective therapeutic options in sepsis, and ATM as a potential molecular target in inflammation-driven conditions.

### DETAILED DESCRIPTION OF THE INVENTION

To identify small molecules that simultaneously inhibit the secretion of TNF and IL-1β, we performed a chemical screen using -2320 compounds. We identified 45 leading candidates (Figure 1A and Table I) that inhibited both cytokines.

**Table I**

| **List of drug candidates with a simultaneous effect on TNF and IL-1b secretion sorted according to the TNF score** | | | |
|---|---|---|---|
| **ID** | **Compound** | **Z score TNFa** | **Z score IL1b** |
| **1505708** | **Epirubicin hydrochloride** | **-4,474857631** | **-1,212875226** |
| 300037 | Crassin acetate | -4,448741528 | -2,126298968 |
| 1501193 | Erysolin | -4,442389411 | -1,162701697 |
| 1504079 | Tomatine | -4,440219344 | -2,626087357 |
| 330001 | Dactinomycin | -4,13760156 | -1,187827575 |
| **1505483** | **Doxorubicin** | **-4,071115136** | **-2,412138965** |
| 200007 | Gambogic acid | -3,862359373 | -1,529656531 |
| 200090 | Obtusaquinone | -3,829374842 | -1,532189516 |
| 200022 | Aklavine hydrochloride | -3,511681732 | -1,408917561 |
| 1504181 | Pristimerin | -3,474946679 | -0,952562058 |
| 1505955 | Colistin sulfate | -3,457325884 | -0,918619053 |
| 1504082 | Dihydrocelastrol | -3,387513735 | -1,102010407 |
| 1505908 | Mangostin trimethyl ether | -3,162805301 | -1,634164354 |
| 1504218 | Acrisorcin | -3,10607388 | -0,959612692 |
| 300549 | Acetyl isogambogic acid | -3,056795183 | -1,48116787 |
| 201522 | Gambogic acid amide | -3,013926221 | -1,517154207 |
| **1500223** | **Daunorubicin** | **-3,007093086** | **-0,995459172** |
| 201604 | Pyrromycin | -2,980583694 | -1,456377283 |
| 1500260 | Pyrithione zinc | -2,951809549 | -1,872292546 |
| 201664 | Celastrol | -2,941287145 | -1,499560887 |
| 1500319 | Gramicidin | -2,91855912 | -1,84556962 |
| 1503006 | Benzyl isothiocyanate | -2,908582737 | -1,034306584 |
| 1503904 | Patulin | -2,874616362 | -0,902703858 |
| 1503640 | parthenolide | -2,842267433 | -0,921962794 |
| 100005 | Anthothecol | -2,834385142 | -1,868706598 |
| | | | |

| ID | Compound | Z score TNFa | Z score IL1b |
|---|---|---|---|
| 100009 | Cedrelone | -2,817231786 | -1,852572153 |
| 1504098 | Phenothrin | -2,810792588 | -0,983918754 |
| 1505438 | Hydrocortisone valerate | -2,794271243 | -2,722889618 |
| 1504240 | 1,4-naphthoquinone | -2,77797604 | -1,349015947 |
| 1505450 | Prednisolone hemisuccinate | -2,72427413 | -2,095888301 |
| 1500315 | Gentian violet | -2,696385799 | -1,835724332 |
| 310010 | Helenine | -2,683894042 | -0,943744357 |
| 310035 | Sanguinarine sulfate | -2,651620816 | -1,160017114 |
| 1SO2074 | Alexidine hydrochloride | -2,648173863 | -0,94122L729 |
| 1503278 | Mitoxanthrone hydrochloride | -2,562729474 | -1,158920135 |
| 1505723 | Betamethasone acetate | -2,560814606 | -0,950513609 |
| 100146 | 7-desacetoxy-6,7-dehydrogedunin | -2,517707805 | -1,789647817 |
| 1503432 | Mepartricin | -2,504992413 | -1,081865327 |
| 201524 | Dihydrogambogic acid | -2,488781431 | -1,48996453 |
| 1505722 | Desoxymetasone | -2,417754689 | -0,995122645 |
| 1505726 | Desonide | -2,297715909 | -0,945977097 |
| 1500521 | Pyrvinium pamoate | -2,268148981 | -1,683811286 |
| 1505168 | Ethacridine lactate | -2,218841761 | -1,389229184 |
| 1501149 | Ritodrine hydrochloriide | -2,149761571 | -1,268303571 |
| 1505125 | Alclometazone dipropionate | -1,999200408 | -0,922964076 |

This inhibitory effect was dissociated from cytotoxicity of the compounds tested On THP-1 cells. Among them we found 3 representatives of the anthracycline family of chemotherapeutic agents (epirubicin, doxorubicin and daunorubicin). To validate the effect of the top candidate in the inhibition of IL-lβ and TNF secretion we treated THP-1 cells with increasing concentrations of epirubicin and found the half-inhibitory concentration (IC50) to be 0.40µM for IL-1β and 0.50µM for TNF, (Figure 1B). In addition, we have tested the effects of additional members of the anthracycline groups of drugs (daunorubicin and doxorubicin) identified in the primary chemical screen and found similar results to those obtained for epirubicin (Figure 5). We conclude that the group of anthracyclines, not epirubicin alone, inhibits the secretion of TNF and IL-1β, suggesting that is not a specific effect of epirubicin but a general property of the group of anthracyclines possibly related to their DNA damage properties.

To study the effects of epirubicin treatment on THP-1 and *E*. *coli* stimulated THP-1 cells, we performed an unsupervised clustering analysis of genome-wide mRNA expression profiles. We found a common epirubicin gene expression signature when comparing drug-treated and untreated samples, that includes both up and down-regulation of a subset of genes (Figure 1C, cluster Epi1 and Figure 1C, clusters Epi2-4, respectively). Gene ontology (GO) classification reveals enrichment for genes involved in the DNA damage response, cell cycle control and apoptosis. The changes in gene expression seen in the major clusters of upregulated (Figure 1C, clusters Ecoli1 and Ecoli3) or down-regulated (Figure 1C, clusters Ecoli2) genes by *E*. *coli* are eliminated in epirubicin treated cells. These gene clusters are significantly enriched in GO categories and pathways related to immune and inflammatory responses. To validate the effects of epirubicin observed in microarrays, we used RT-qPCR to measure the relative expression of genes identified in the major bacteria-induced clusters (Figure 1D).

We choose the cecal ligation and puncture (CLP) mouse model to investigate the in vivo effects of epirubicin. In CLP, sepsis results from a polymicrobial infectious abdominal origin, leading to bacteraemia and a systemic inflammatory response¹⁵. We have adjusted its severity to a high-grade sepsis, where at least 80% of C57BL/6 mice die within 48hrs after the initial procedure. Under these conditions, epirubicin administered i.p. in a saline solution (PBS) at the time of CLP and again 24hrs later in a total of 1.2µg/g of mice body weight reproducibly and significantly increased the survival of C57BL/6 mice subjected to CLP by nearly 80%, without the use of any antibiotic (Figure 2A). Intravenous injection of epirubicin produced similar results (data not shown). The in *vivo* protective effect is not restricted to epirubicin, as we have also observed comparable protection when using doxorubicin and daunorubicin in similar conditions (Figure 6). As for the in vitro results, this suggests that also in vivo, antracyclines, not epirubicin alone, are protective in sepsis. One week after epirubicin, administration, mice are not immunocompromised as they clear a secondary intranasally infection with the muHV-4 virus similarly to control mice (Chora and Marques et al., unpublished). We determined that the protection was not due to an antibiotic effect of epirubicin, given that the drug also completely protected C57BL/6 mice from death in the LPS model of lethal septic shock (Figure 2B) and epirubicin-treated and non-treated mice showed similar numbers of blood circulating bacteria after the CLP procedure (Figure 2C). Therefore, epirubicin confers host tolerance to infection without increasing bacterial killing. Our findings are counter-intuitive as anthracyclines have been shown to induce acute inflammation when injected in the abdomen^{16,17}. However, the drug concentrations utilized in these studies were at least 10-ford higher than those used here. By using lower concentrations we may reduce the cytotoxicity of these drugs (and the resulting releasing of pro-inflammatory DAMPs by dying cells), and possibly reveal additional pharmacological effects mediated by the target cells.

We next investigated the systemic inflammatory response induced by CLP. Twenty-four hrs after the CLP procedure, we observed robust secretion of inflammatory mediators such as TNF, IL-1β, IL-6 and HMGB1 that was dramatically reduced in epirubicin-treated mice (Figure 2D-G). To validate the protective mechanisms induced by anthracyclines we measured serological markers of organ damage in CLP mice in the serum of non-treated mice and compared them with those of epirubicin-treated mice 24 hrs after the initial CLP procedure. Serum concentration of LDH (lung and general cellular damage), CK (muscle), ALT (liver) and Urea (kidney) were reduced to almost basal levels in mice treated with epirubicin *vs* untreated mice (Figure 2H-K respectively), suggesting that anthracyclines have strong protective effects in organ function.

To identify mechanisms of epirubicin protection in CLP, we performed an in *vitro* short hairpin RNA (shRNA)-based screen, focusing on kinases and phosphatases. We found several negative regulators of IL-1β in response to *E*. *coli* challenge, including the Ataxia Telangiectasia Mutated (ATM) and the Checkpoint Kinase 1 (cheek1) genes (Figure 3A and data not shown), both critical mediators of a DNA Damage Response (DDR). ATM is a master regulator of the DDR pathway¹⁰ and was known to be activated by anthracyclines and other DNA damaging agezats¹⁹. Using a phospho-specific antibody against the activated form of ATM, we found that epirubicin, alone or in combination with *E*. *coli* stimulation, triggered the activation of ATM (Figure 3B). *E. coli* stimulation alone was a poor, but reproducible, activator of ATM (Figure 3B). We confirmed these results using immunoblotting (data not shown). *In vitro,* epirubicin was able to decrease the secretion of both IL-1β and TNF, but only the IL-1β down-regulation was dependent on the activation of ATM, which could be rescued by using the ATM specific inhibitor KU-55933 (Figure 3C). Epirubicin-mediated down-regulation of IL-1β, CCL2 and CXCL10 mRNA expression levels (Figure 3D and data not shown) was ATM-dependent, but was ATM-independent in the case of TNF (Figure 3D).

To test the contribution of ATM to the protective effect of epirubicin against severe sepsis, we compared the survival of wildtype (*Atm*^{+/+}) *vs*. ATM-deficient (Atm^{-/-}) mice subjected to CLP and treated with epirubicin. We found that ATM expression is absolutely necessary to mediate the protective effect of epirubicin. (Figure 4A), but is possibly not sufficient. In line with this interpretation, etoposide, known to cause less complex DNA damage, only partially rescues the mortality induced by CLP despite activating ATM-dependent pathways²⁰ (Figure 4B).

We then explored possible mechanisms to explain the protective role of epirubicin in sepsis. We found that, *in vitro*, epirubicin is able to counteract the increase in ROS generated by *E. coli* challenge of THP-1 cells in an ATM-dependent manner (Figure 8). However, mice that are deficient for the nuclear factor (erythroid-derived 2)-luke 2 (NRF2), a master regulator of ROS scavenging, are still protected by epirubicin against mortality due to CLP (Figure 9). Therefore, epirubicin induces an ATM-dependent ROS scavenging response that is largely dispensable for its protective effect in sepsis.

Increased apoptosis of neutrophils can attenuate sepsis pathogenesis²¹. This would be a simple and attractive hypothesis considering that anthracyclines initiate a DDR leading to increased apoptosis if the DNA lesion is too severe for repair²¹. However, our data shows that epirubicin treated mice have higher, not lower, numbers of viable neutrophils in the abdomen, excluding an important role for this mechanism (Figure 7B).

The biogenesis of some miRNAs, including miR-146a (a negative regulator of inflammation²² and a proposed biomarker in sepsis²³), is ATM-dependent²⁴. We therefore compared the survival of wildtype mice with that of miR-146a -deficient mice in the presence or absence of epirubicin. We conclude that the protection given by this drug is dependent on the presence of miR-146a (Figure 10). However, our RT-qPCR analysis of miR-146a expression in either RAW cells (data not shown) or THP-1 cells (Figure 11) does not support a role for epirubicin in the induction of this microRNA. Therefore, direct induction of miR-146a is not the mechanism by which epirubicin protects against the LPS model of septic shock or CLP.

We next explored the requirement for autophagy induction in epirubicin-induced protection. We predicted that epirubicin-induced ATM activation could increase and sustain autophagy because DNA damage is known to induce autophagy²⁵, possibly in an ATM-dependent manner³⁶, and ATM is a negative regulator of mTOR, which is itself, an inhibitor of autophagy^{27,29}. Using FACS analysis, we found that epirubicin induces autophagy in THP-1 cells and that this effect is ATM-dependent (Figure 4C). Pre-treatment with the ATM inhibitor KU-55933 eliminates this effect (Figure 4C). We then compared the survival of wildtype mice with autophagy-deffective (LC3b - deficient) mice in the presence or absence of epirubicin. The data shows that the autophagy pathway is required for the in *vivo* effect of epirubicin in sepsis protection (Figure 4D). We conclude that the protective effect of epirubicin in sepsis is, at least in part, due to the ATM-dependent induction of autophagy.

Finally, we studied the therapeutic window of epirubicin in mice. When given alone, epirubicin confers strong protection at the time of the procedure or until 3hrs after the initiation of CLP. When administered only 6hrs after CLP, epirubicin quickly loses its protective effect. However, if given in combination with meropenem, even if this antibiotic is only administered 12hrs after CLP, low dose epirubicin confers complete protection until at least 24hrs after the initial procedure (Figure 4E). Our results suggest that epirubicin, and more generally the group of antracyclines, are very effective at preventing mortality due to sepsis, under conditions that reflect the human standard of care. The 24hr therapeutic window is likely to be sufficient to make this drug useful in the clinic for most patients that are either in the hospital or seek medical attention within the first few hours of symptoms initiation.

### Object of the invention

In this way, the object of the invention is an anthracycline for using in the treatment of sepsis, the anthracycline being selected from epirubicin, doxorubicin and daunorubicin, in particular epirubicin.

Although the antrhracyclines are suitable for the treatment of any kind of sepsis, they are particularly efficient in the treatment of severe sepsis.

### Experimental part

### Methods

### In vitro Chemical screen

THP-1 human monocytes (American Tissue Culture Collection-ATCC TIB-202) were plated in 96 well plates at 10⁶ cell/ml and incubated with each one of ∼2320 compounds included in the Spectrum collection (Microsource Discovery Systems, Gaylordsville, CT) at 10µM for 1 hour. Cells were then challenged with 4% PFA-fixed DH5α *E.coli* at a Multiplicity of Infection (MOI) of 20 bacterial cells per THP-1 cell for an additional 24 hours. The cell supernatants were collected and IL-1β and TNF cytokines quantified by DAS-ELISA, using Human IL-1β/IL-1F2 DuoSet^{®} and Human TNF DuoSet^{®} (R&D Systems ^{®}), respectively, according to company's protocol.

### Transcriptome profiling

Raw microarray data was normalized using the aroma.affimetrix package ²⁹ implemented in R. Clustering analysis was performed with Multiple ExperimentViewer MeV4 _7_3 ³⁰ on a subset of 3513 genes selected from the full array dataset for an absolute log2 expression of at least 7 in two (out of eight) arrays. To further reduce the number of genes to cluster, a variance filter was applied to select the 50% highest SD genes. After mean centering the data by gene/row mean, a hierarchical clustering analysis was performed using Euclidean distance metrics. Gene ontology and pathway enrichment analysis was performed on selected clusters of genes using the program GO-Elite (http://www.genmapp.org/go_elite/go_elite.html) ³¹.

### The RNAi Consortium RNAi library

Detailed description of the RNAi Consortium (TRC) lentiviral RNAi library used in this study was originally described in ³² (see www.broad.mit.edu/rnai/trc/lib for additional details).

### shRNA-based screen

We generated a working subset of The RNAi Consortium (TRC) shRNA lentiviral vector library ^{33,33} that allows for the silencing of most of the genes that are either human kinases or phosphatases. This subset was composed of 1440 individually arrayed lentiviral shRNA vectors targeting ∼700 genes, after selecting the most efficient shRNAs (two on average) based on available silencing efficiency data from the Broad Institute of MIT and Harvard. THP-1 cells were plated in 96 well plates at 10⁶ cell/ml and infected with shRNA-expressing lentivirus. 48 hrs later infected cells were selected with puromycin. After the 3 days of selection, plates were duplicated. One of the plates was used to measure the cell number using Alamar Blue^{®} cell viability assay (Invitrogeia^{®}), according to manufacturer's instructions. In the other plate, cells were stimulated with 4% PFA-fixed DH5α *E.coli* at a Multiplicity of Infection (MOI) of 20 bacterial cells per THP-1 cell. Twenty-four hours after stimulation, cell supernatants were collected and IL-1β and TNF cytokines quantified by DAS-ELISA. All data values from IL-1β and TNF secretion assays were normalized by dividing the amount of IL-1β and TNF in the conditioned media 24, 12, 8, 6, 4 or 2 hrs after *E*. *coli* stimulation by the number of cells in each well and then by the average concentration per cell of the plate. Results were logarithmic natural transformed. Scores were sorted in ascending order and graphed. We calculated 1.5 SDEVs above and below the mean to identify the genes that changed IL-1β and TNF secretion when silenced. The same approach was used to identify the compounds that changed IL-1β and TNF secretion. The selected genes were submitted to two or more rounds of phenotypic validation.

### RT-qPCR

Total RNA was extracted with TRIzol (Invitrogen) and reverse transcribed with Superscript II Reverse Transcriptase (Invitrogen). Quantitative PCR reaction was performed with Power SYBRgreen (Applied Biosystems) on Rotor-Gene 6000 real-time thermal cycler (Corbett Life Science /QIAGEN). Primer sequences were retrieved from Primer Bank (http://pga.mgh.harvard.edu/primerbank/) or designed using Primer-BAST (http://www.ncbi.nlm.nih.gov/tools/primer-blast/). Values were formalized to GAPDH.

### Assessment of autophagy and ROS content

THP-1 cells were stimulated as described earlier. Autophagy was assessed by incubating cells with Cyto-ID™ Green detection Reagent (Enzo Life sciences) according to the manufacturer's instructions. Generation of cellular free radicals was assessed by incubating cells for 20 minutes with 0.5mM of the broad free radical probe 5-(and 6)-chloromethyl-2'7'-dichlorodihydrofluoscein diacetate acetyl ester (CM-H2DCFDA; Molecular Probes). Fluorescence was measured by flow cytometry, and data analyzed using FlowJo software.

### Mice

Animal care and experimental procedures were conducted in accordance with Portuguese and US guidelines and regulations after approval by the respective local committees (Instituto de Medicina Molecular and Instituto Gulbenkian de Ciência). All mice used were 8-12 weeks old. Mice were bred and maintained under specific pathogen-free (SPF) conditions. C57BL/6 and C57BL/6 *ATM*^{*-*/*-*} were obtained from the Instituto Gulbenkian de Ciência (a kind gift from Dr. Vasco Barreto). C57BL/6 *Nrf2*^{*-*/*-*} mice were provided originally from the RIKEN BioResource Center (Koyadai, Tsukuba, Ibaraki, Japan)³¹ and subsequently at the Institute Gulbenkian de Ciência. Cecal Ligation and Puncture was performed as described previously¹⁵.

### Colony-Forming Units Assay

Peritoneal fluid was obtained by peritoneal lavage with 5 ml of sterile PBS (Sigma) and blood was collected by cardiac puncture. Serial dilutions of blood and peritoneal lavage were immediately plated on Trypticase Soy Agar II plates supplemented with 5% Sheep Blood. CFUs were counted after 12 hours of incubation at 37°C.

### Stainings and flow cytometry,

Peritoneal infiltrating leukocytes were obtained by lavage with 5 ml of sterile ice-cold PBS (Sigma), washed and blocked with mouse Ab anti-FcγIII/II (clone 93) receptor mouse Ab diluted in PBS containing 2% FCS (v/v) for 20 minutes at 4°C. Surface markers were detected by incubating for 30 minutes at 4°C with mouse Ab anti-CD4 (clone GK1.5), -CD8 (clone 53-6.7), - CD19 (clone 6D5), -Ly-6G (clone 1A8) (all Biolegend) and - neutrophils monoclonal antibody (clone 7/4) (Abcam). Dead cells were excluded by co-staining with propidium iodide. Total cell number was determined by flow cytometry using a fixed number of latex beads (Beckman Coulter) co-acquired with a pre-established volume of the cellular suspension. For phospho-ATM intracellular staining, stimulated T14P-1 cells were washed and fixed with ice-cold methanol. Mouse Ab anti-phosphoATM pS1981, clone 10H11.E12 (IgG1k) (Rockland) was incubated for 60 minutes at room temperature followed by an incubation of secondary Ab conjugated with Alexa 488 (Molecular Probes). Fluorescence was measured by flow cytometry, and data analyzed using FlowJo software.

### Immunoblotting

Mouse phospho-ATM (4526, Cell Signaling, Danvers, MA, 1:1000 dilution) and rabbit total ATM (2873, Cell Signaling, Danvers, MA, 1:1000 dilution) antibodies were used overnight at 4°C. Primary antibodies were detected using peroxidase conjugated secondary antibodies (1h; RT) and developed with SuperSignal chemiluminescent detection kit (Pierce, Carcavelos, Portugal).

### References

1. Cohen, J. The immunopathogenesis of sepsis. Nature 420, 885-891 (2002).
2. Doi, K., Leelahavanichkul, A., Yuen, P.S. & Star, R.A. Animal models of sepsis and sepsis-induced kidney injury. J Clin Invest 119, 2868-2878 (2009).
3. Cavassani, K.A., et al. TLR3 is an endogenous sensor of tissue necrosis during acute inflammatory events . J Exp Med 205, 2609-2621 (2008).
4. Medzhitov, R. Origin and physiological roles of inflammation. Nature 454, 428-435 (2008).
5. Medzhitov, R. & Janeway, C.A., Jr. Innate immunity: the virtues of a nonclonal system of recognition. Cell 91, 295-298 (1997).
6. Janeway, C.A., Jr. & Medzhitov, R. Innate immune recognition. Annu Rev Immunol 20, 197-216 (2002).
7. Sims, G.P., Rowe, D.C., Rietdijk, S.T., Herbst, R. & Coyle, A.J. HMGB1 and RAGE in inflammation and cancer. Annu Rev Immunol 28, 367-388 (2010).
8. Takeuchi, O. & Akira, S. Pattern recognition receptors and inflammation. Cell 140, 805-820 (2010).
9. Bone, R.C., Sibbald, W.J. & Sprung, C.L. The ACCP-SCCM consensus conference on sepsis and organ failure. Chest 101, 1481-1483 (1992).
10. Rittirsch, D., Flierl, M.A. & Ward, P.A. Harmful molecular mechanisms in sepsis. Nat Rev Immunol 8, 776-787 (2008).
11. Suffredini, A.F. & Munford, R.S. Novel therapies for septic shock over the past 4 decades. JAMA 306, 194-199 (2011).
12. Angus, D.C. & Wax, R.S. Epidemiology of sepsis: an update. Crit Care Med 29, S109-116 (2001).
13. Ulloa, L. & Tracey, K.J. The "cytokine profile": a code for sepsis. Trends Mol Med 11, 56-63 (2005).
14. Martin, G.S., Mannino, D.M., Baton, S. & Moss, M. The epidemiology of sepsis in the United States from 1979 through 2000. N Engl J Med 348, 1546-1554 (2003).
15. Rittirsch, D., Huber-Lang, M.S., Flierl, M.A. & Ward, P.A. Immunodesign of experimental sepsis by cecal ligation and puncture. Nat Protoc 4, 31-36 (2009).
16. Krysko, D.V., et al. TLR-2 and TLR-9 are sensors of apoptosis in a mouse model of doxorubicin-induced acute inflammation. Cell Death Differ 18, 1316-1325 (2011).
17. Sauter, K.A., Wood, L.J., Wong, J., Iordanov, M. & Magun, B.E. Doxorubicin and daunorubicin induce processing and release of interleukin-1beta through activation of the NLRP3 inflammasome. Cancer Biol Ther 11, 1008-1016 (2011).
18. Ciccia, A. & Elledge, S.J. The DNA damage response: making it safe to play with knives. Mol Cell 40, 179-204 (2010).
19. Siu, W.Y., et al. Topoisomerase poisons differentially activate DNA damage checkpoints through ataxia-telangiectasia mutated-dependent and -independent mechanisms. Mol Cancer Ther 3, 621-632 (2004).
20. Montecucco, A. & Biamonti, G. Cellular response to etoposide treatment. Cancer Lett 252, 9-18 (2007).
21. Garrison, S.P., et al. The p53-target gene puma drives neutrophil-mediated protection against lethal bacterial sepsis. PLoS Pathog 6, e1001240 (2011).
22. Taganov, K.D., Boldin, M.P. & Baltimore, D. MicroRNAs and immunity: tiny players in a big field. Immunity 26, 133-137 (2007).
23. Wang, J.F., et al. Serum miR-146a and miR-223 as potential new biomarkers for sepsis. Biochem Biophys Res Commun 394, 184-188 (2010).
24. Zhang, X., Wan, G., Berger, F.G., He, X. & Lu, X. The ATM kinase induces microRNA biogenesis in the DNA damage response. Mol Cell 41, 371-383 (2011).
25. Rodriguez-Rocha, H., Garcia-Garcia, A., Panayiotidis, M.I. & Franco, R. DNA damage and autophagy. Mutat Res 711, 158-166 (2011).
26. Chen, L.H., et al. Autophagy inhibition enhances apoptosis triggered by BO-1051, an N-mustard derivative, and involves the ATM signaling pathway. Biochem Pharmacol 81, 594-605 (2010).
27. Alexander, A., et al. ATM signals to TSC2 in the cytoplasm to regulate mTORC1 in response to ROS. Proc Natl Acad Sci U S A 107, 4153-4158 (2010).
28. Alexander, A., Kim, J, & Walker, C.L. ATM engages the TSC2/mTORC1 signaling node to regulate autophagy. Autophagy 6(2010).
29. Bengtsson, H., Irizarry, R., Carvalho, B. & Speed, T.P. Estimation and assessment of raw copy numbers at the single locus level. Bioinformatics 24, 759-767 (2008).
30. Saeed, A.I., et al. TM4 microarray software suite. Methods Enzymol 411, 134∼193 (2006).
31. Salomonis, N., et al. Alternative splicing in the differentiation of human embryonic stem cells into cardiac precursors. PLoS Comput Biol 5, e1000553 (2009).
32. Moffat, J., et al. A lentiviral RNAi library for human and mouse genes applied to an arrayed viral high-content screen. Cell 124, 1283-1298 (2006).
33. Root, D.E., Hacohen, N., Hahn, W.C., Lander, E.S. & Sabatini, D.M. Genome-scale loss-of-function screening with a lentiviral RNAi library. Nat Methods 3, 715-719 (2006).
34. Itoh, K., et al. An Nrf2/small Maf heterodimer mediates the induction of phase II detoxifying enzyme genes through antioxidant response elements. Biochem Biophys Res Commun 236, 313-322 (1997).
35. Shannon Reagan-Shaw, et al. Dose translation from animal to human studies revisited. The FASEB Journal●Life Sciences Forum 22, 659-661 (2007).

## Claims

1. An anthracycline for use in the treatment of sepsis wherein the anthracycline is selected from epirubicin, doxorubicin and daunorubicin and is administered once as soon as possible after diagnosis and again 24 h later, in the same dosage, in a total dose of 1.2 *µ*g/g of body weight for epirubicin and daunorubicin or in a total dose of 1.0 *µ*g/g of body weight for doxorubicin.

2. The anthracycline for use according to claim 1, which is epirubicin.

3. The anthracycline for use according to any one of the claims 1 to 2, in the treatment of severe sepsis.

## Patentansprüche

1. Ein Anthrazyklin für die Behandlung von Spesis, worin das Anthrazyklin aus Epirubicin, Doxorubicin und Daunorubicin gewählt wird und so früh wie möglich nach Diagnose und erneut, mit derselben Dosis, 24 Stunden danach verabreicht wird, in einer Gesamtdosis von 1,2 µg/g des Körpergewichts für Epirubicin und Daunorubicin oder in einer Gesamtdosis von 1,0 µg/g des Körpergewichts für Doxorubicin.

2. Das Anthrazyklin für Behandlung nach Anspruch 1 ist Epirubicin.

3. Das Anthrazyklin für Behandlung nach eines der Ansprüche 1 bis 2 bei der Behandlung schwerer Sepsis.

## Revendications

1. Une anthracycline destinée à être utilisée dans le traitement de la septicémie où l'anthracycline est choisie parmi l'épirubicine, la doxorubicine et la daunorubicine et est administrée une fois dès que possible après le diagnostic, puis une autre fois 24 heures après, à la même dose, dans une dose totale de 1,2 µg/g de poids corporel pour l'épirubicine et la daunorubicine ou dans une dose totale de 1,0 µg/g de poids corporel pour la doxorubicine.

2. L'anthracycline destinée à être utilisée selon la revendication 1, qui est l'épirubicine.

3. L'anthracycline destinée à être utilisée selon l'une quelconque des revendications 1 et 2, dans le traitement de la septicémie grave.
